# EUROPEAN PATENT APPLICATION

(11) **EP 1 902 741 A2**
(43) Date of publication of application: **26.03.2008**
(21) Application number: 07018537.6
(22) Date of filing: 20.09.2007
(51) Int. Cl.: A61M 16/00, A61M 16/10

(54) **Respirators for delivering clean air to an individual user**

(30) Priority: 20.09.2006 US 533529
(71) Applicant: Next Safety, Inc., West Jefferson, NC 28694 (US)
(72) Inventor: Hebrank, Jack, Durham, NC 27712 (US); Hunter, Charles Eric, Jefferson, NC 28640 (US); Lemahieu, Edward, San Jose, CA 95125 (US); Hartley, Chris, Boone, NC 28607 (US); Criss, Ron, West Jefferson, NC 28694 (US); Hunter, Jocelyn, Jefferson, NC 28640 (US); McNeil, Laurie, Chapel Hill, NC 27599-3255 (US); Jones, Charles, Jefferson, NC 28640 (US); Duvall, Lyndell, Fleetwood, NC 28626 (US); Wetzel, Paul, Jefferson, NC 28640 (US); Ballou, Jr. Bernard L., Raleigh, NC 27614 (US)
(74) Representative: Rehberg Hüppe + Partner

(57) **Abstract**

Disclosed are personal respirators and clean air systems.

## Description

This application claims priority to and is a continuation-in-part of copending U.S. application serial number 11/268,936, filed November 8, 2005; of copending U.S. application serial number 11/317,045, filed December 23, 2005; of copending U.S. application serial number 11/412,231, filed April 26, 2006; of copending U.S. application serial number 11/434,552, filed May 15, 2006; and of U.S. application serial number (official filing receipt not yet received), filed July, 17, 2006 (client reference "CIP 4"), each of which is incorporated herein by reference in their entireties. In addition, this application claims priority to copending U.S. provisional application serial number 60/796,368, filed May 1, 2006, which is also incorporated herein by reference in its entirety.

### FIELD OF THE INVENTION

The invention relates to respirators. More particularly, the disclosed respirator relates to a respirator that cleans air, usually atmospheric ambient air, at the point of respiration and delivers the clean air to an individual user.

### BACKGROUND

Human beings have inhabited the earth for more than ten thousand years. Only in the last 200 years, starting roughly with the Industrial Revolution, have the respiratory systems of human beings been continuously exposed to heightened levels of airborne pollutants. For people who live in urban or suburban areas today, there is no escape from airborne contaminants such as particulate exhaust, ozone, dust, mold and the many other pollutants in outdoor city air. Furthermore, studies show that in the housing of even the most affluent city dwellers, indoor air can be, and often is, dirtier than the air outside. As a practical matter, people who live in cities, whether in developed or developing nations, and regardless of their affluence, have been and continue to be without any defense against the ravages of dirty air. Additionally, rural areas in much of the world have air pollution conditions that are as problematic as those found in cities, due in part to the location of fossil fuel power plants and, in developing nations, the widespread presence of factories and motor vehicles without any effective pollution controls. The human respiratory system simply has not had time to develop a defense against today's air contamination and, as a result, public health suffers in the form of various pulmonary diseases, including an alarming increase in the incidence of asthma, as well as other diseases such as cancer, pulmonary fibrosis, colds and flu viruses, and other respiratory diseases. Surprisingly, in the twenty-first century there is no effective, widely adopted defense against polluted or contaminated air, in fact, no defense at all for ordinary citizens going about their daily activities. To the extent that systems are currently in use to deliver clean air to individuals, such systems are primarily limited to use in connection with workers exposed to hazardous airborne contaminates in the workplace *(e.g.,* asbestos, coal dust, spray paint).

As mentioned above, the cleaning of air in indoor residential and commercial settings is, as a general rule, wholly inadequate to significantly reduce airborne contaminates. The typical whole-room air filtration system utilizes particle filters in the flow conduits that supply air to the room, most typically, in an HVAC system. Such HVAC filtration systems sometimes, but rarely, include other means for purifying the air, for example, catalytic surfaces that remove certain chemicals, or a radiant energy source, for example ultraviolet radiation, that kill the RNA and DNA of certain airborne pathogens. Of course, the most heavily filtered whole-room systems are found in clean rooms, such as those utilized in the electronics industry.

It should be noted that filtration alone in clean rooms is not sufficient to maintain low air particle densities. Extremely rapid and complete air changes are also required. This requirement is caused by the internal generation of particles by human movement. Therefore the only mechanism for providing clean air to humans is at the point of respiration.

To provide clean air at the point of respiration, one approach is to passively filter such as by a surgeon's face mask. For purposes of this application the term "passive" refers to masks that are unpowered and therefore do not include an air mover such as a blower. Such masks, and similar cloth masks, are extremely leaky, due to the poor seal between the face and the mask. Estimated filter efficiency of these masks is about 90 percent for 300 nanometer particles and smaller. Furthermore, it is well known that these masks are hot and uncomfortable because they trap exhaled moisture and because the user must exert additional effort to breathe to overcome the pressure drop across the mask. Furthermore, due to their passive nature and the risk of pulling contaminated air in through the sides of the mask, these types of masks require careful fitting and are leaky for people with facial hair or whose facial contours otherwise do not conform to the mask. It will be appreciated that passive devices, being unpowered, do not provide a positive pressure and flow of air.

Another system in use for providing clean air to industrial workers in the workplace is the Positive Air Pressure Respirator (PAPR), manufactured by 3M, which includes a loose fitting hood or full face mask. The PAPR system has a high leak rate that requires significant air flow and power consumption beyond the capability of any easily carried battery pack. Thus, AC sources or very large and heavy batteries typically power them. The complexity of design makes them unsuitable for widespread use by ordinary citizens.

Another system in current use is the Continuous Positive Airway Pressure(CPAP) system, manufactured by several medical suppliers such as Puritan Bennet and Respironics, which is a pressurized mask that typically covers the nose and mouth and is designed with sufficient resilience and strength to keep the system air flow hoses open in sleeping situations and prevent the mask from collapsing or breaking in persons suffering from sleep apnea. Furthermore, the CPAP system typically delivers air to the patient at a substantial pressure above atmospheric, adjustable from 15 to 30 centimeters of water. Because of the high pressure drops and resultant energy demands of these systems, they are typically plugged into fixed power sources. Although portable blowers exist for ease of travel, the higher pressure increases the power requirements significantly. Some of the CPAP units have particulate filters, and some do not. These devices are not designed to support inhalation rates of active, awake adults.

In addition to the examples identified above, the prior art includes other face masks with various forms of filters, face masks connected to chemical air filtration systems and face masks connected to compressed air cylinders for underwater diving and firefighting.

A comprehensive review of the literature and simple observation reveals the lack of any point-of-respiration air cleaning apparatus that filters substantially all particulates and biological pathogens down to 25 nanometers or below, or for that matter, up to or above several microns. Further, even at particle sizes of 300 nanometers (above) the best filter efficiency is only 99.97%, which in the case of influenza A sub-types, certain of which have caused pandemics killing more than 50million people (which range from 80 to 120 nanometers), the filter is wholly ineffective and would not prevent such a pandemic again. Even more importantly, many portable point of respiration devices fail to provide flow rates at or above 350 standard liters per minute (slm). A certain percentage of humans under routine work conditions have average respiration rates approaching under continuous flows 350 slm. Because prior devices have limited flow capacity, according to a NIOSH sponsored study, humans wearing masks suffered significant drops in blood oxygen saturation; these drops were so substantial that human participants in the study were withdrawn from further participation. Without sufficient flow, there is substantial risk that wearers' blood oxygen saturation levels would reach unsafe levels resulting in significant hemodynamic compromise including death. This also precludes safe use of all tight fitting masks by humans, particularly those with breathing disorders, such as asthmatics, lung cancer recovery patients, pulmonary fibrosis, emphysema and others of acute or chronic respiratory sensitivity. In particular, devices used in biocidal applications, such as a pandemic, currently do not have the airflow capacity to support humans working in high stress situations that would be typical and, furthermore, would not allow the wearer to sneeze without removing the mask, thereby risking exposure to pathogens.

Furthermore, certain devices are offered for sale to wearers with flexible hoods where there is no ability to measure negative pressures relative to atmospheric and sound an alarm in case a negative pressure is detected, which would occur under a number of failure mechanisms such as filter blockage, blower failure, battery discharge, or failure. These closed hooded devices are found in biocidal applications.

Furthermore, devices do not exist that have a form-factor and weight required for widespread consumer acceptance and use. Also, the larger and heavier devices known in the art have an intrinsically high cost further limiting the ability of these devices to be utilized in many critical applications where negative pressure relative to atmosphere would cause contaminated air to diffuse into the mask.

The present application proposes a lightweight, portable air-cleaning respirator for use by ordinary people in everyday activities, preferably a respirator built upon a platform that can be adapted to clean air in many different settings. These respirators can be used anytime an individual wishes to breathe highly purified air, for example, for one or two hours while relaxing in the evening, during commuting hours, when outdoors, or on high-ozone or high pollen days, etc.

### SUMMARY

Disclosed are respirators and clean air systems. In one embodiment, an exemplary respirator apparatus includes a housing, an air mover mounted in the housing, the air mover being operable to generate an air stream and having an input and an output, a particle filter mounted in the air stream, and a supply hose operably connected at one end to the housing, wherein the housing with its air mover is implemented as a reusable portion of the system, while the particle filter and supply hose are implemented to be removable from the reusable portion. In one embodiment, the respirator is configured to provide an air supply of approximately 200 standard liters per minute (slm) with an air reserve reservoir configured to provide a user of the system with ample filtered air for large instantaneous demand without requiring the blower and other system components be sized to meet such demand. In one embodiment, particle filter comprises a wet-laid glass media. In one embodiment, the air mover is an impeller mounted in the housing, the air mover being operable to generate an air stream at a rotational speed of at least 5,000 rpm.

### BRIEF DESCRIPTION OF THE DRAWINGS

The disclosed methods and respirators can be better understood with reference to the following drawings. The components in the drawings are not necessarily to scale.
Figure 1 shows an air supply system of an earlier-filed application from which priority is claimed,
Figure 2A and B show a three-dimensional representation of one embodiment of a system of the disclosed respirator,
Figure 3 shows a side view of various parts of the system of Figure 2,
Figures 4A and 4B show one embodiment of a hose of the disclosed respirator,
Figures 5A-C shows an embodiment of a hose of the disclosed respirator,
Figures 6A and B show various views of one embodiment of a particle filter of the disclosed respirator,
Figures 7A and B show different views of another embodiment of a particle filter of the disclosed respirator,
Figures 8-9 show further embodiments of a particle filter of the disclosed respirator,
Figure 10 shows a side view of one embodiment of a public air supply adaptor of the disclosed respirator,
Figure 11 show a side view of an embodiment of a public air supply adaptor of the disclosed respirator,
Figure 12 shows an embodiment of a face mask of the disclosed respirator,
Figure 13 shows an embodiment of a pair of goggles of the disclosed respirator,
Figure 14 shows one form of a seal between a hose and a filter cartridge,
Figure 15 shows one form for a seal between air flow connectors,
Figure 16 shows a skin-like flexible face mask,
Figure 17 shows a three-dimensional view of one embodiment of a pleated particle filter,
Figure 18A and B show front and top views of one embodiment of a spacer for a pleated filter,
Figure 19 shows a cross section of one embodiment of a filter cartridge connected to a hose,
Figure 20 shows a hose connected to a mask extension,
Figure 21 shows the seal between a mask and a user's face,
Figure 22 shows a three-dimensional view of one embodiment of a face mask of the disclosed respirator,
Figure 23 shows a three-dimensional view of another embodiment of a face mask of the disclosed respirator,
Figure 24A, B, C show side, top, and end views of one embodiment of a PPR of the disclosed respirator, and
Figure 25 shows one embodiment of a bag for carrying a respirator system of the disclosed respirator.
Figure 26 shows an exemplar embodiment of a respirator system that can utilize a blood oxygen saturation sensor attached to the wearer's finger or other body part with a wired or wireless transmission to control flow rates, add O₂ or sound alarms for mask removal or filter change.

### DETAILED DESCRIPTION

While the disclosed respirator will be described more fully hereinafter with reference to the accompanying drawings, in which aspects of the preferred manner of practicing the disclosed respirator are shown, it is to be understood at the outset of the description which follows, that persons of skill in the appropriate arts can modify the disclosed respirator herein described while still achieving the favorable results of this disclosed respirator. Accordingly, the description which follows is to be understood as being a broad, teaching disclosure directed to persons of skill in the appropriate arts, and not as limiting upon the disclosed respirator.

Disclosed are portable positive pressure respirators (PPRs) that supply an ample amount of filtered air to the user while being constructed in such a way that the size, weight, and cost of the system makes it suitable for routine use during everyday activities. An "ample amount" of filtered air means air sufficient to support a user's air intake needs during use. For example, the disclosed PPRs provides air at a flow rate of at least approximately 350 standard liters per minute (slm). The disclosed respirator effectively removes particles greater than 60 nanometer while still allowing high airflows required for human respiration. The disclosed respirator therefore seeks to capture the market that includes people who wish to protect their lungs in a comfortable way from dust, particulates, pollen, mold, viruses, bacteria, carpet particulates and other airborne matter.

One embodiment of the disclosed respirators provides clean air at the point of respiration to a degree that is comparable to the ultra stringent standards set in the semiconductor industry (Class I clean room manufacturing semiconductor devices with 65-nanometer line widths). Furthermore, these standards are far higher than those found in any hospital environment, where more than 100,000 citizens die yearly from infections acquired within the hospital.

A survey of current Powered Air-Purifying Respirator (PAPR) systems on the market today reveals that most supply filtered air at the rate of 200 slm or less with a few models from various manufacturers demonstrating flow rates up to 450 slm in favorable conditions. However, none of these models provides highly purified air, 99.999% filter efficiency or better for particles or microorganisms from 25 to 300 nanometers. Current literature and recent research findings indicates that there is a wide range of respiration rates to meet depending upon the respiratory capacity of the individual and the physical activity level of the wearer. It is suggested that PAPR devices supplying less than 200 slm are inadequate for many individuals at moderate exertion levels and even tacit acknowledgement by National Institute for Occupational Safety and Health (NIOSH) that it may be desirable to set minimum flow requirements that are greater than those typically provided by the current art. Additionally, research has indicated that under very strenuous activity *(e.g.,* a fireman carrying a heavy pack up a flight of stairs) that it is necessary to provide flow at up to 600 slm to maintain adequate blood oxygen levels. Therefore the disclosed PPR, in one embodiment, supplies approximately 350 slm as a standard that, unlike a majority of the current art, will be adequate for a vast majority of the human population for most activities, even including moderate exercise. Additionally, the disclosed PPR in another embodiment will provide approximately 600 slm that, unlike any known current device, will supply highly filtered air at a rate which is suitable for even the most robust individuals under the most strenuous work environments.

In searching the literature, the typical filtration efficiency employed in similar PAPR units supplied in the industry is High-Efficiency Particulate Air (filter) (HEPA)-grade (99.97% efficient at 300 nm). In order to be effective against particles such as the H5N1 virus, the disclosed PPR exhibits filtration efficiency that, at a minimum, is Ultra Low Penetration Air (filter) (ULPA)-grade (99.999% efficient at Most Penetrating Particle Size (MPPS) and rated air velocity). The filter efficiency is determined in large part by two factors. One of these factors is the performance and construction (grade) of the filter material itself. At least two grades of wet-laid glass media manufactured by Lydall Filtration/Separation in Rochester, NH meet these requirements (6650 and 6850 grades).

The other factor, given a typical filter media, is the speed at which the air and the particles suspended in the air strike the filter as they pass therethrough (typically referred to in the industry as "face velocity"). It has been demonstrated and is well known within the filtration industry that filtration efficiency is increased with decreasing face velocity. The major physical principle involved in this behavior is the kinetic energy of the particles contained within the air (proportional to the square of velocity). One of the principle features of the disclosed PPR, aside from the use of highly efficient filter media, is the use of large filter surface area to minimize face velocity and thereby maximize filter efficiency. For example, the filtering surface area of the filter is at least approximately 100 square inches in size. In this way, it is possible to achieve filter efficiencies of 99.9999% or greater for particles as small as 25 nanometers (the size of the human cold virus).

The portability aspect of the disclosed PPR requires a portable power supply with sufficient energy storage and acceptable weight. In order to ensure that sufficient energy storage to power the device can be attained without excessive weight, a blower with low power consumption is employed to move the air through the system. Currently, impeller or blower technology exists that can move the desired airflow at the required pressures while consuming less than 8W. Additionally, centrifugal blowers have the property of using less energy when airflow is less than their rated flow. Therefore, a blower rated at 8W used in a system such as the disclosed PPR can be operated for up to 4 hours on a battery with a capacity of 2 Ampere-hour (Ah) or greater. These blowers are readily available at rated voltages of 12 V and up. Conveniently, current technology Li-ion cells such as those currently used in laptop computers and cell phones are employed in the disclosed PPR and meet the desired energy density described above. Four 18650 Li-ion cells in series deliver a rated voltage of about 14.4 V maximum, and have capacities of 2.4 Ah or greater (about 34 Watt hours). This allows run times with two of these battery packs beyond 8 hours with a total battery weight of approximately 400 g (14 oz). By contrast, the NiMH battery pack for the 3M Powerflow^{™} system weighs about 800 g (28 oz).

As with weight, acceptable overall size (physical volume) of the disclosed PPR is a factor in providing a portable, comfortable, attractive unit that is acceptable to widespread consumer use. To a certain extent, the volume of the unit is dictated by the size of the filter. By specifying flow rate and filtration efficiency, the filter area is thereby determined. Additionally, the properties of the filter media at a given area and flow rate subsequently define a set pressure drop across the media itself which must be overcome by the impeller blower. Thus, indirectly, setting flow and filter efficiency values dictates the pressure/flow characteristics of the blower. Pressure drops in the hose and other components of the system can also be taken into account (pressure drop in the hose is directly proportional to flow and inversely proportional to the fourth power of the hose diameter).

For the pressure and flow levels on the low end of the performance range in which the disclosed respirator operates, the blower will typically sustain 350 slm flow at a pressure of 1.2 in H₂O. On the high end of the range, the blower can sustain approximately 600 slm at up to 2.5 in H₂O. However, a single blower with performance characteristics on the high end of the range can be used for any performance level between 350 slm and 600 slm. An examination of the physical principles involved with developing pressure using a centrifugal blower show that the biggest factor in developing pressure with such an impeller is the speed at which the outside edge of the impeller travels. In turn, this is determined by the rotational speed of the fan (rpm) and its diameter. Increasing either the rpm or diameter of the impeller directly affects the pressure that the impeller is capable of developing.

Notwithstanding design details that can take advantage of the impeller pressure capability *(e.g.,* sealing structures, interior contours, etc.), an impeller of 10 cm diameter is capable of producing about 1.2 in H₂O (the low end of the performance range of the current invention) at about 5000 rpm. To generate 2.5 in H₂O the same impeller would have to be driven at more than 10,000 rpm. Similarly, a 7-cm diameter fan would have to be driven at between 7,000 rpm and 14,500 rpm. Using a smaller impeller reduces the overall size of the disclosed PPR; however, this advantage is offset for the disclosed PPR in terms of cost. An impeller/blower that currently has an acceptable cost and expected life at 5,000 rpm cannot be operated at 10,000 rpm due to the increased demands on motor bearings and the tendency toward vibration, particularly with larger impellers at higher rpm levels. Therefore, a blower which meets the size and performance requirements for the high (600 slm) end of the operating range is currently not optimal at the low end of the range due to much higher cost of the technology and materials required for a high rpm blower.

The above discussion therefore points toward at least two embodiments rather than a single preferred embodiment. In this way, the added cost and complexity of a unit capable of operating at 600 slm can be avoided in the embodiment intended to address the 350 slm range.

One embodiment can be employed for the lower end of the performance range, such as a large-diameter impeller (10 cm) operating at a moderate rpm value (5000 rpm). The filter media can be either of a number of grades. For instance, a filter of 2800 cm² of **filter media exhibiting 99.999% efficiency at, for example, 100nm particle size under this application** could be employed to meet the requirements of ULPA level filtration and minimize system volume while keeping pressure drop to a minimum to take full advantage of the pressure flow characteristics of the low speed impeller. An example of an acceptable filter is the Lyndall 6650 filter. Alternatively, this impeller could be combined with a filter using 3700 cm² of a media **exhibiting 99.9999% efficiency at 60nm particle sizes or below** that would increase filtration efficiency to a point well beyond the ULPA level while increasing the system volume and maintaining flow. An example of an acceptable filter is the Lyndall 6850 filter. With a 25 mm i.d. smooth bore hose, the flow for the above embodiment would be near 350 slm.

One embodiment that can be used for the high end of the operating range is the smaller (7 cm) high-speed (15,000 rpm) impeller. As with the above embodiment, the filter and media can be any of a number of grades as described above to balance desired efficiency (ULPA to 99.9999% efficiency) and system size/volume. With a 25 mm i.d. hose the flow for this embodiment of the system would be approximately 600 slm.

A further embodiment would incorporate a smaller (5 to 7 cm) high speed impeller which is optimized for cost. In combination with a filter media with the highest possible filtration efficiency, the PPR would be able to operate throughout the desired flow range at a cost point comparable to the embodiment mentioned for the low range above.

One feature of the disclosed respirator is that it accommodates the unique limitations of portability by conserving energy. The disclosed respirator nevertheless supplies sufficient air for respiration at a positive pressure and at air flows which prevent users from experiencing a reduction in saturated blood oxygen levels due to wearing the respirator. This air flow is achieved in one embodiment with an air mover in the form of a centrifugal blower coupled with a particle filter having a filter surface area that is large enough to provide adequate air flow to the user and to provide low filter face velocities required to achieve high filter efficiencies without requiring high power to the blower, e.g. 3000 square centimeters or more for a filter optimized for 60-nm particle size. By providing a filter surface area that is substantially larger than is known in the current art, the disclosed respirator provides for an efficient use of energy. High flow rates are achieved through the use of a larger diameter air supply hose, *e.g.,* 20-25 mm outside diameter. Alternatively, where aesthetics are of greater concern and in settings where demand is relatively low, flow rates can be moderated by making use of a hose that is smaller than any comparable PAPR mask supply hose known in the art *e.g.,* 10-16 mm outside diameter hose. Nevertheless, by appropriately adjusting blower power, blower pressure is preferably controlled to ensure adequate face mask pressure to achieve leakage flow during the passive state and thereby always ensuring a positive pressure inside the mask.

Thus another feature of the disclosed respirator is the maintenance of a positive pressure in the mask ranging from a very slight positive pressure (for energy conservation reasons, especially when a narrower supply hose is being used) to large positive pressure (in certain applications and when consumption is very high, which is then preferably associated with a large diameter hose to minimize pressure losses in the hose). Thus, in spite of the energy conserving aspect of the disclosed respirator, the disclosed respirator nevertheless proposes a system that ensures a continual positive pressure above atmospheric in the mask. Thus, in the case of a poor seal between mask and face, air will flow out and not in. The mask typically includes an outlet valve that is biased-closed but can be maintained in a slightly open position during passive phase. The mask can also include an inlet valve but can instead be provided without an inlet valve. When the user inhales, the blower supplies the required air at a pressure greater than the pressure drop through the filter and any inlet valve so that the pressure in the mask remains above atmospheric pressure and the user is protected from drawing outside air into the mask.

Thus the basic system can leak out (through the valve or through the side of the mask) an amount of air that is a small fraction of the air supply capability of the blower, thereby preventing contaminated air from flowing into the mask, while allowing sufficient reserve air for breathing and minimizing the power consumed by the pump. It should be noted that the shaft power requirements for centrifugal pumps decreases as fluid flow through the pump decreases. However, as noted above, one aspect of the disclosed respirator includes actively controlling power to the air mover, *e.g.*, the pump to further decrease power during lower demand phases, such as during passive state or exhaling and during sedentary periods and for people consuming less air, *e.g.,* children.

Thus in order to further conserve energy, power to the blower can be adjusted manually or automatically to take into account different air flow demands for different activities, different users, and depending on whether the user inhales, exhales or is in a passive state between exhaling and inhaling. Airflow demand can be determined by measuring pressure in the face mask using a pressure sensor or by measuring flow rate in the hose using a flow rate sensor. Alternatively the flow or pressure can be monitored as a function of blower rotational speed (rpm). Thus a feature of the disclosed respirator is the efficient use of energy by controlling the power to the blower so as to take account of different airflow demands. The system can include a pressure sensor in the mask, and the signal from the pressure sensor can be used to control power to the air mover. In another embodiment an air flow sensor can be used for sensing air flow in the hose and using the signal from the air flow sensor to control power to the air mover.

The pressure or flow sensor, possibly in communication with a microprocessor, will preferably switch on a light emitting diode and in certain embodiments actuate an audible alarm to indicate to the user that the system pressure is below atmospheric, e.g. due to clogging up of the filter or inoperability of the blower. Additionally, the light emitting diode and/or audible alarm can also be initiated by a pre-defined increase in power to the blower. The user can then replace the filter (which can be housed in a filter cartridge) or the blower can be replaced at the user's convenience depending on which element needs replacing. Since the disclosed respirator envisages providing a filter quality and filter area combination that allows the user to breathe without the benefit of a filter, even a partially clogged filter that requires replacement will allow the user to continue breathing with or without the help of the blower for short periods of time without requiring the mask to be removed. Once the filter cartridge or blower is replaced a reset button can be activated to reset the alarm.

Instead of a sensor to automatically compensate for changes in demand, the system can simply include a manual actuator for adjusting power to the blower as required by the user.

In addition to controlling the power to the blower (automatically or manually), the system can include a manually or electronically actuated valve mounted in the air stream, e.g. in the hose or housing or face mask. In the automatic embodiment, the valve is controlled by the signal from a pressure sensor and a closed loop control circuit will adjust the valve to control the airflow. Thus, the system can be implemented to maintain a slightly positive air pressure in the mask.

Another feature of the disclosed respirator is the placement of the particle filter (also referred to as the main filter). The particle filter preferably is a 100 nm or below *(e.g.,* 60 nm) filter that is preferably mounted downstream of the blower. Filters are normally placed at the air intake to an air mover *(e.g.,* blower) to facilitate filter replacement and keep all debris out of the system. However, this disclosed respirator's preferred placement of the filter downstream of the air mover permits simple filter replacement with the replacement or cleaning of the mask/tube system and simplifies blower construction by not requiring an airtight seal between the blower intake and environmental air. This simplifies the blower construction, therefore reducing cost and allowing access and cooling of motor electronic control circuits. Additionally, placing the blower upstream of the 100 nanometer or lower filter ensures that in the event of a malfunction vaporized polymers will not reach the user. The filter assembly can also have a prefilter to remove large particles that would otherwise increase the pressure drop across the primary filter. Again, in order to avoid debris from the pre-filters from reaching the user, the pre-filters are preferably mounted upstream from the main or particle filter.

The mask or hose can include a second inlet with a filter for providing air to the user in case the air supply from the housing is interrupted.

In addition the mask can include a channel or conduit to the outlet valve to maintain a channel of clean air. The channel or conduit can be arranged in the wall of the mask or along an inner or outer surface of the face mask. The air outlet can include a split manifold in which air is split into two or more channels exiting the mask as two or more outlet ports. Each outlet port can include its own outlet valve or the inner portion of the air outlet prior to the split can be provided with an outlet valve. The two or more channels or conduits provide for a more balanced and aesthetically pleasing mask and helps dampen or eliminate the inadvertent opening of the outlet valve when the user encounters high velocity air such as encountered when riding a bicycle or facing the wind.

The mask can further include a backup air outlet provided with a filter to filter out particles and pathogens from the user's exhaled air.

The face mask can be made wholly or partially from transparent material so that at least the mouth of the user is visible. The mask wall thickness can be reduced over the mouth portion to improve communications by the user.

The system can further include eye protection in the form of goggles or glasses, which can be separate from the breathing mask, especially in biocidal applications. The use of a pair of glasses or goggles ensures that the user's eyes are protected from exposure to air that might be contaminated by pathogens or other types of particles.

As discussed above, the disclosed respirator also considers the diameter of the hose and the use of the hose with a large area filter and appropriate adjustment of blower power to achieve the desired air flow rates. The flow of air between the air mover and the inlet of the face mask can be provided by a supply hose, which can take the form of a flexible tube or comprise a flattened profile conduit with one or more air flow channels extending through the conduit. To avoid being crushed the wall thickness of the hose can be ribbed or be of sufficient thickness to avoid occluding the one or more air channels if a user leans against the supply hose. As indicated above, where aesthetics are of more concern than flow rate for individuals requiring lower continuous air flows *(e.g.,* children), the hose can be of a smaller diameter than the prior art devices of 18-20 mm diameter. Alternatively, a larger, *e.g.,* 25mm hose may be substituted where maximizing the airflow is more important than, for example, aesthetics. A baffle can be included in the mask to divert the incoming air stream associated with high airflow rates.

A ribbed hose can be divided into sections interspersed by un-ribbed sections, *e.g.*, ribbed sections that are 4-6 inches long to allow it to be readily cut to the desired length.

Preferably the hose is made of a highly flexible material to make it less unwieldy, and for aesthetic reasons, the hose can be made of transparent material and can have the same styling as the mask to appear as if integrally made with the mask, *e.g.,* be smooth as the mask. Also, the hose is preferably made of low cost materials for disposability as is discussed in greater detail below.

Utilizing centrifugal type air movers at power levels that allow reasonable system run-time with batteries of reasonable size and weight for portable human use, the minimum hose diameter for a 2.5 foot hose at airflows at or above 350 slm is 20mm and preferably equal to or greater than 25mm. Reasonable power consumptions are maintained according to the disclosed respirator by providing a larger area filter than has been done before in the art. The hose is made of a highly flexible material that has a smooth surface on the interior and provides support through an exterior wire or other means to prevent the hose from changing shape.

The placement and connection of the main filter into the system is another feature of the disclosed respirator. In particular, the air mover can be mounted in a housing having an air inlet and an air outlet. A pre-filter can be provided upstream of the air mover, *e.g.,* at the air inlet of the housing and/or can be contained in the filter housing on the output side of the blower. The particle filter (main filter), which typically is a high quality filter with the capability of filtering out 60 nanometer or 100 nanometer particles, can be mounted in or partly in the housing and can define a hose connector for connecting the air supply hose that leads to the face mask. The hose can instead be connected directly to the housing. The particle filter is preferably mounted downstream of the air mover to define a clean air environment downstream of the filter. This has the advantage that the air mover need not be located in the clean air environment. Instead of being mounted in or partially in the housing, the filter can be mounted in the hose or in or on the face mask. The filter can be comprised of one or more filter elements in a housing. The housing can be arranged to receive a circular "screw on" or clip on filter cartridge or enclosure or a rectangular or square clip on filter cartridge. The filter cartridge can also house one or more prefilters or air purifying devices, *e.g.,* a course particle filter, or devices for removing ozone SO₂ or NO₂, etc. Preferably the high quality particle filter, *e.g.,* a 60 nm or 100 nm ULPA filter is located on the downstream side of the pre-filters to prevent any particles which escape the pre-filter from contaminating the air stream. As indicated above, the pre-filter can have a function other than simple filtration, *e.g.,* for removing certain gases such as CO. Instead of being mounted in the same cartridge as the main particle filter, the pre-filter can be separately mounted in the air stream and can be implemented in different forms, *e.g.,* by making use of an impregnated sponge, ring layer, or surface coating of manganese dioxide or copper oxide catalyst to filter out CO. A solid ring layer impregnated with calcium hydroxide or calcium silicate solids can remove sulfur dioxide and nitrogen dioxide. Ozone, in turn, could be removed using a honeycomb ceramic, such as the Honeycycle ZG impregnated with active carbon, or by use of a gettering material such as titanium, aluminum, or other materials that react with ozone to form more stable oxides in fiber, sheet, particulate, or other suitable forms. The ozone gettering and catalyst materials may be imbedded in the filter material such as manganese oxide fibers with boro-silicate fibers in a latex matrix to provide an ozone catalyst and a filter, or which could be used as a prefilter. Materials that catalyze the recombination of ozone to O₂ such as manganese dioxide and copper oxide can be used in fiber, sheet, particulate, or other suitable forms.

Yet another feature of the disclosed respirator is the configuration of the main filter and optimizing the filter efficiency. One advantage of a square or rectangular filter cartridge is that it makes use of rectangular filters thereby optimizing filter material usage by avoiding wastage between filters that are cut out of a filter sheet. The filters themselves are preferably pleated to increase the surface area. As one feature of the disclosed respirator, the pleats can have rounded ends to allow higher airflow through the filter. In order to maintain the shape of the pleats one or more spacers can be included, which can shape or form the ends of the pleats and space the pleats evenly apart. The spacers can be implemented in a serpentine fashion so as not to obstruct airflow. Instead of round or square configuration filters, the filter can be an annular (donut-shaped) filter that wraps around a blower in cases where the air from the blower is expelled radially outwardly. The housing may, in such a filter arrangement form an annular channel around the filter, with an outlet connectible to a hose.

Yet another feature of the disclosed respirator is the provision of a cost effective breathing apparatus that makes use of low cost elements. In particular certain elements can be designed to have a limited life expectancy that is significantly shorter than the life expectancy of prior art devices. Thus, the filters or the filter cartridges are preferably arranged to be easily removable to allow them to be disposed of and replaced with new filters or filter cartridges. As an alternative, the entire face mask with the hose and filter can be arranged to be disposable. On the other hand other elements of the system, such as the housing with its blower and power supply can be designed with a long life expectancy so they can be re-used.

The system typically includes a power supply or energy source, which can take the form of at least one battery or multiple batteries mounted in a cartridge, or a re-chargeable battery pack receivable in a compartment in the housing. In the case of a battery pack, the pack can include a plug, *e.g.,* a plug on a lead that is receivable by a complementary socket mounted in the housing. In the case of a battery pack, the pack typically includes electrical contacts and the housing preferably includes a compartment with complementary contacts. In certain embodiments, the housing is adapted to receive two battery packs or battery cartridges to provide redundant energy sources and facilitate hot swapping of energy sources. The two energy sources can be of the same or different size. For certain end uses, the system can instead or in addition include an AC adapter to allow the system to be powered off an AC outlet or to facilitate charging of batteries or battery packs. The AC adaptor can be mounted inside the housing.

The housing with its air mover and power supply and filter (also referred to herein as a PPR) can be housed in a carrying bag that serves other functions. For example the PPR can be housed in a bag containing a laptop computer, in which case it can be powered via the USB port of the computer or the PPR power supply can be used to power the laptop. Likewise the computer and the PPR can share the same power supply.

The power sharing connection between the PPR and other devices is handled by appropriate power conditioning circuits. For instance, energy from the USB port on a laptop computer is used to power the PPR unit by stepping up the USB voltage using a DC to DC step-up converter as is known in the art. Likewise, other electronic devices such as cellular telephones, global positioning satellite receivers, music and video downloading and playing devices (*e.g.,* iPOD^{®}) and DVD players can share the same power supply or can power each other.

The housing with its air mover and energy source can be implemented as a reusable portion of the system, while the filter, pre-filter, hose and face mask can be implemented to be removable from the reusable portion, and be disposable. As such, the hose and possibly also the face mask can be made more cheaply (thinner material and/or cheaper material) than would be the case if the hose and face mask were intended to have a similar life expectancy as the reusable portion.

In order to ensure that the particle filtering capabilities of the high quality filter are not compromised, the hose is connected at each of its two ends by means of at least one seal that prevents ingress of particles. In the case of disposable portions being connected together, the connection or seal can take the form of a permanent seal or connection, *e.g.*, a thermal seal or adhesive, while any connections between disposable and re-usable portions can be implemented using a friction fit connection that can further include a gasket, such as a non-porous foam gasket or one made of the high quality filter material to define a separable connection. For greater integrity the seal can include a redundant layer seal material. This can take the form of two or more ring layers of sealant to avoid the integrity of the seal being compromised by mechanical stress, manufacturing defect, etc. The separable connection can include a clamp to secure the two portions to each other.

The housing can be implemented as a belt mounted arrangement, wherein the housing fits into a fanny-pack type arrangement or fits into a bag that is securable to a belt. The housing can be implemented to have a designer apparel outer skin or covering. In the case where the housing is received in a bag or pack, the bag or pack can be implemented as a fashion item, *e.g.*, with designer apparel trade dress. Instead, or in addition thereto, the system can include swatches or covers, preferably comprising designer apparel swatches that at least partially cover the housing or bag.

More generally, the PPR system can fit into a specifically designed or a standard bag such as a designer purse, a mountaineer backpack, a computer carrying case. The PPR system can be designed of such length, width, and height to fit into predefined pockets of existing fashion accessories. Additionally, such accessories have been selected as to provide sufficient space to store a mask, several filters, and an optional collapsible hose or several sets of child size masks and hoses with filters such as for a family.

In particular, market acceptance for the product is enhanced by the integration of the PPR unit with accessories such as but not limited to, designer handbags, hip bags or back-packs. Thus, the PPR could be easily carried in a carrying device of the user's choice with maximum versatility, or in a handbag/hip bag/backpack specially created to enhance the function of the PPR unit. The implementation of system components in various colors and patterns will allow for numerous personalization options. As a business strategy, the disclosed respirator contemplates providing the system as a fashion accessory and providing the ability to customize, or differentiate this product from others which are designed principally with utility in mind. The small physical size, minimal weight, and convenient shape of the PPR system are required for its use in such an embodiment.

One or both of the housing and hose (or as indicated above, the filter cartridge) can include means for reducing at least one of ozone, carbon monoxide, sulfur dioxide and nitrogen dioxide in the air entering the housing. The means for reducing ozone can comprise an ozone catalyst for splitting off an oxygen atom from the ozone molecule or a material that reacts with ozone that forms a stable oxide. The means for reducing carbon monoxide can include a carbon monoxide catalyst that adds an oxygen atom to the CO to form CO₂. The ozone catalyst can comprise active carbon. The carbon monoxide catalyst can comprise titanium, manganese dioxide or copper oxide. In addition or alternatively, sulfur dioxide and nitrogen dioxide can be removed using calcium hydroxide or calcium silicate solids, or a wetted lime slurry in a sponge. At least one of the carbon monoxide catalyst and ozone catalyst can comprise a coating on at least part of the inner wall of the housing or the hose or both the housing and the hose.

Instead of mounting the filter cartridge in or partially in the housing and connecting the second end of the hose to the filter cartridge, the particle filter can be mounted in or on the hose (e.g. at the first end of the hose connecting to the face mask) or in the face mask. In addition, the face mask can include a second particle filter in flow communication with the air outlet for filtering particles from the exhaled air.

In the case where the particle filter is connected to the second end of the hose, the filter can include a second connector for connecting the filter to at least one public air supply system or to an adaptor for connecting to at least one public air supply system. The adaptor can be provided with a plurality of connectors for connecting to a plurality of public air supply systems. The connector for connecting the hose to at least one public air supply system or to the adaptor or for connecting the adaptor to the at least one public air supply system, can include at least one friction fit connection, each of which can include a gasket and the at least one friction fit connection can include a clamp for securing the connection. The gasket can be made from a non-porous foam material or of a filter material.

As indicated above, the particle filter can be designed to be a short-term filter with a defined useful lifespan, *e.g.,* one day (16 hours) or in another embodiment for a period of one week (with 7 16-hour days).

Still further according to the disclosed respirator, there is provided a pair of eye protecting goggles, comprising a lens portion and a peripheral gasket with air vents, wherein the air vents are provided with HEPA or ULPA filters. For purposes of this disclosed respirator the term "lens" refers simply to the clear portion of the goggles and does not suggest that the "lens" has convex or concave surfaces. The gasket can comprise two or more rows of material arranged one behind the other to define multiple seals or a seal with multiple ribs and troughs. The material of the gasket can include non-porous foam material or HEPA or ULPA filter material. The filters in the air vents can be arranged to be replaceable. The peripheral gasket can be attached to the lens portion or the goggles can include a frame to which the gasket is attached.

Further according to the disclosed respirator there is provided eye protection goggles comprising a lens portion without a peripheral frame but simply provided with a peripheral gasket, wherein the gasket includes multiple rows of sealing material formed integrally to define a single gasket or formed as separate gaskets arranged parallel to each other.

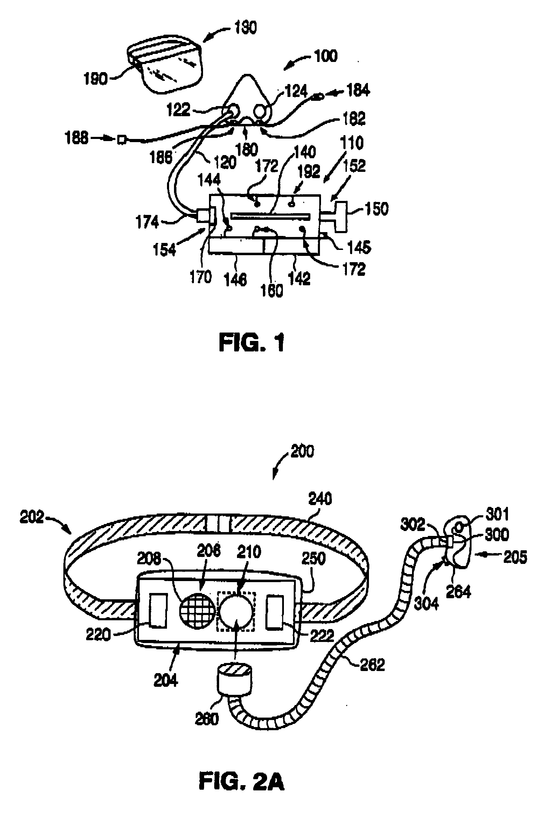

In earlier filed application serial number 11/412,231 entitled "Air Supply Apparatus" filed April 26, 2006 an air supply system is described with respect to Figure 1 (which is included in this application as Figure 1). The system of Figure 1 includes a filter and a means for moving the air, *e.g.*, a pump, fan, or blower, as well as means for controlling either the flow rate of the air stream or the air pressure. The system is contrasted over the prior art on the basis that the prior art devices make use of constant high flow rates with small area filters, which prevents use of filter materials better than HEPA grade due to the large pressure drop. The applicants system shown in Figure 1, on the other hand, makes use of a controlled air flow system to avoid these drawbacks. In the embodiments described in applicant's application 11/412,231 means are described for killing or destroying organic contaminants in the air stream *e.g.,* by radiating the air stream with UV radiation, however, as described in that application the air supply system is equally applicable for use without any means for killing or destroying biological contaminants. Since the disclosed respirator deals with a system that includes an air mover mounted in a housing with a particle filter, preferably located at the downstream side of the air mover, and a face mask connected to the housing or filter by means of a delivery tube or hose, without any UV radiation source, it is instructive to consider the embodiment of Figure 1 again since the disclosed respirator builds on and incorporates many of the features of the earlier applications.

As discussed in the earlier application 11/412,231and as shown in Figure 1, the system of Figure 1 includes a face mask 100 connected to a kill chamber 110 by means of a flexible delivery tube 120. It will be appreciated that the term "kill chamber" was chosen here for the reason that the chamber includes a UV light source for destroying DNA or RNA of air-borne pathogens. In an embodiment where the housing serves only to support the air mover, power supply and filter, the simple term chamber or housing is more appropriate. For purposes of this application, embodiments without any UV light source will be referred to simply as involving a housing. The face mask 100 includes a one-way intake valve 122 and a one-way exhaust valve 124. The face mask 100 fits over a person's nose and mouth with the exhaust valve 124 sending the exhaled air into the atmosphere. The intake valve 122 allows the person to inhale clean air. The one-way valves 122, 124 ensure that the person breathes clean air while eliminating the used air to the atmosphere. The valves 122, 124 can be simple flapper valves, over center flapper valves, or electrically actuated valves. In one embodiment, the valve open area was chosen to correspond approximately to the cross-section of a human trachea (about 3-5 cm²). The delivery tube 120" which is preferably made of a flexible material is chosen to have a similar cross-section (3-5 cm²). In a preferred embodiment, the mask 100, valves 122, 124, and delivery tube 120 are designed to be removable from the kill chamber 110 to facilitate washing, and are preferably made of a dishwasher safe material. By providing for quick release connectors or otherwise providing connectors that allow the kill chamber, delivery tube or hose, and face mask to be readily separated from each other, the various parts allow for easy exchange of worn out parts or use of components from another apparatus. In one embodiment, the apparatus includes eye protection such as glasses or goggles, or a flip-down transparent visor as indicated by reference numeral 130. The visor 130 of this embodiment includes a heads-up display and a receiver 190 for receiving external feed for displaying information on the display 130. The receiver 190 can be a wireless receiver *e.g.*, a WiFi receiver for receiving wireless Internet feed or cached content information feeds. In the embodiment shown, an air mover in the form of an air pump 170 is included in the chamber 110 to provide a positive pressure within the mask 100 thereby ensuring that the surrounding air is not inadvertently drawn into the mask 100 along its sides where it abuts the user's face. The pump 170 also serves to ease the inhaling process by providing an airflow toward the mask 100. One such pump is a centrifugal pump, *e.g.* REF 100-11/12 sold by EBM-Papst, Inc. of Farmington, CT. Instead of pushing air directly to the mask, the pump, in another embodiment my supply a supply tank which then feeds the face mask via an appropriate regulator at the mask or tank.

In this embodiment, the kill chamber 110 has an internal volume corresponding approximately to one human breath of an adult under moderate exertion. (The typical breath of a resting adult is about 0.5 liter and under moderate exertion volumes will typically increase to 1 and 1.5 liters for a typical adult.) However, as is discussed in greater detail below, the disclosed respirator will be based on a variable demand approach in which pressure or flow rate is monitored and power to the air mover is adjusted to maintain a constant pressure as demand changes. In fact, in other embodiments the chamber volume is specifically chosen to be smaller than an average breath of a typical user of the apparatus. In the Figure 1 system the kill chamber 110 is tubular in shape with a diameter of approximately three inches (3") and six to eight inches (6-8") in length, and a UV light source 140 is mounted in the chamber 110. In this case a mercury vapor lamp is used as the UV light source, and the lamp is protected in a quartz sleeve to reduce the likelihood of breakage. Also, a sensor 172 is included to monitor the output of the mercury vapor lamp and close a valve 174 to the mask 100 if the lamp stops radiating. The UV light source in this embodiment is powered by means of a power source that, in this embodiment, comprises a battery pack 142. The power source 142 can include a DC to AC converter to facilitate the provision of 120 volts AC or more for powering a mercury vapor lamp from a battery such as a 10 volt DC battery. It will be appreciated that the power source will include appropriate ballasting circuitry. In the case of LEDs being used as the UV source, the power source will provide the appropriate LED current by means of an appropriate DC voltage converter or through the use of optimized circuitry for LEDs as produced by MAXIM. The battery pack constituting the power supply 142 in this embodiment is packaged integrally with the chamber and includes a charger for the battery pack. However, it will be appreciated that the battery pack could also be separately housed and carried, for example, on a user's belt. It will be appreciated that not only the kill chamber with its sensors and battery pack are preferably carried separately from the mask, but any other elements that are not required to be on the mask 100 could also be carried separately from the mask, *e.g.,* in a backpack, shoulder bag, etc. Thus, for example any cell phone, AM/FM radio, walkie-talkie, or visor information receiver could be housed and carried in a backpack with the kill chamber 110. The disclosed respirator provides a number of additions and variations over the system described with respect to Figure 1.

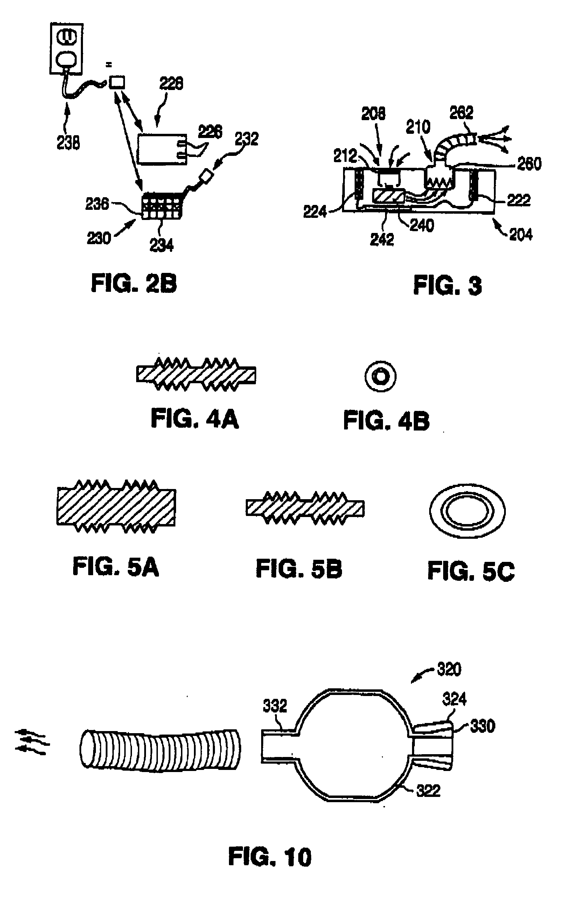

Figures 2A and 2B show a three dimensional view of a basic system of the disclosed respirator in the form of a portable, battery operated respirator system 200 for delivering clean air to an individual user. The respirator system 200 in one embodiment includes a re-usable portion 202 and a disposable portion 205 that is removably connected to the re-usable portion 202. The re-usable portion 202 comprises a housing 204 having an air inlet 206 with a screen or grid 208, and an air outlet 210. The air inlet in this embodiment is provided with a pre-filter 212 (shown in Figure 3). Two power supply or energy source compartments 220, 222 are formed in the housing 204. As shown in Figure 3, the housing 204 houses a blower 206 and a power supply described in greater detail with respect to Figure 2B. As discussed in more detail below, the housing can be carried by a user in conjunction with a fanny pack 250, bag, or the like. The respirator 200 also includes a removable portion (washable and/or disposable) that includes a particle filter 260 (e.g. ULPA or HEPA), a delivery hose 262 and a face mask 264 that includes a mask portion for covering the mouth and nose, which in this embodiment has an inlet valve 302 and an outlet valve 304.

In this embodiment, the blower takes the form of an eight-watt centrifugal blower connected to a 35 to 70 watt-hour rechargeable battery. Actual power consumption at low flow rates will be less than at high flow rates. The blower 206 has a peak pressure at zero flow of about 1.7 inch H₂O and can sustain 350 slm (12 cfm) with a pressure of about 1.2 inches of water. The particle filter 260 in this embodiment is housed in a filter cartridge as is described in greater detail below, and is in the form of an ULPA pleated filter pack that is located just downstream of the blower 206. The pressure drop across the ULPA filter is about 1.0 inches H₂O at 350 slm (linear relationship between flow velocity and pressure drop). The flexible delivery hose or tube 262 connects the air mover (in this case blower 206) to the face mask 264. The one-way inlet valve 302 is chosen as a "zero resistance" valve (0.1 inches H₂O at 350 slm), and the outlet valve 304 is also one way and opens at about 0.5 inch H₂O.

The ULPA particle filter 260 is a submicron filter that has a filter efficiency better than 99.99% for 0.1 micron particles.

In operation the blower pushes air through the filter 260 to the mask 264. At least one prefilter (also described in greater detail below) can be included in the same filter cartridge as the ULPA filter and can include a low quality particle filter for removing very large particles from the air before they reach the ULPA filter. With the user neither inhaling nor exhaling, the pressure in the mask will be about 0.5 inch H₂O (i.e., near the outlet valve actuation pressure of 0.5 inch H₂O).

As the user inhales the peak inhale velocity can be up to 350 slm requiring a blower output of 350 slm or greater. At 350 slm the blower delivery pressure will be about 1.2 inches H₂O. Mask pressure will be blower pressure minus about 1.0 inches H₂O pressure drop in the filter, and about 0.2 pressure drop in the hose and inlet valve. Thus, during an inhale mask pressure remains positive relative to atmospheric pressure until the maximum blower output of 350 slm is exceeded.

As the user begins to exhale, the mask pressure rises above 0.5 inch of H₂O. This causes the mask inlet valve (if utilized) to shut so that blower output flow becomes negligible and blower power consumption drops to about 4 watts automatically since the blower experiences less resistance or load. In this regard, the blower motor preferably is speed controlled so that motor RPM remains relatively constant as the motor load changes. Above 0.5 inch H₂O the mask outlet valve opens so that outlet pressure drop in the valve will be about 0.5 to 1.0 inches H20 as the exhale flow rate goes from 10 to 100 slm.

As the user ends the exhalation, the mask pressure will drop to about 0.5 inch H₂O and the outlet valve will close. As exhale pressure drops below 0.4 inches H₂O the inlet valve will open supplying blower air to the user.

The above description is modeled on the typical respiration of a young adult male (Guyton, Medical Physiology) which is about a 500 mL inhale happening about 12 times a minute or once every five seconds. Peak inspiration rate might be estimated as 0.5 liter in 0.1 to 1.0 second or about 30 to 300 slm average inhalation rates with instantaneous values somewhat higher.

As mentioned above, the blower motor automatically uses less power when flow rate is reduced. In order to further conserve energy, the blower motor in one embodiment is actively controlled to change the current according to the demand. Thus as demand increases due to inhalation current to the blower can be increased, whereas current is decreased during lower demand *e.g.,* exhalation and passive state between inhalation stage and exhalation stage. This is achieved in one embodiment by means of a pressure sensor, *e.g.* a pressure sensor mounted in the mask. During passive state, when there is only leakage flow and therefore little pressure drop in the hose (essentially only the 0.1 inch H2O drop across the inlet valve, the mask pressure will be 0.9 inch H2O since the blower generates a pressure of 1 inch H2O.
During inhaling, as air flow rises possibly to about 300 or 350 slm, the blower supplies about 1.2 inch H2O. At this flow rate, pressure loss through the hose and the filter is significant and is about 0.9 to 1.0. Thus, with the 0.1 inch H2O drop across the inlet valve (if employed), the pressure in the mask is significantly lower at about 0.2 to 0.3 inches H2O meaning that demand has gone up. During exhalation the pressure in the mask is above 0.5 inch H2O due to exhaled air (the inlet valve, if used, is closed by the exhaled air and the outlet valve is opened and remains so as long as pressure in the mask stays above 0.5 inch H2O. Thus pressure in the mask is high implying a low demand. Thus the two high pressure, or low demand stages (passive state and exhalation) can be measured and used to control the motor by reducing the current to the motor. In one embodiment this is done by a microprocessor (not shown). Instead of monitoring pressure in the mask, the flow rate in the hose can be monitored by a flow rate sensor. At high flow rates, when the user inhales and the inlet valve opens, is associated with high demand. On the other hand a low flow rate, *e.g.*, when there is only leakage flow during passive state or when there is no flow or very little flow due to the inlet valve being closed or almost closing due to exhale back pressure, the demand is low and current to the blower can be reduced.

As shown in Figure 2B, in this embodiment the compartment 220 is provided with electrical contacts for contacting complementary contacts 226 on a rechargeable battery cartridge 228. The compartment 222, in turn, is sized to receive a battery pack 230 and is provided with a socket (not shown) for receiving a complementary plug 232. The battery pack in this embodiment comprises a plastic holder 234 for supporting 4 cylindrical batteries 236. The purpose of having two energy sources is in order to provide a backup if one fails and also to allow replacing of energy sources one at a time without losing power (hot swapping). It will be appreciated that both energy source compartments could instead be for battery packs or both for battery cartridges and the two packs or two cartridges could be the same size or of different size. The power supplies or energy sources 228, 230 serve as energy sources for an air mover in the form of a centrifugal blower 240 and to power control circuitry 242. The blower 240 (which in other embodiments can be replaced by a fan or a pump) generates an air stream from the air inlet 206 through the pre-filter 312, to the air outlet 210. It will be appreciated that the use of a battery pack or battery cartridge provides for portability. By making use of a rechargeable battery cartridge 228 or rechargeable batteries in the battery pack 230 the power sources can readily be recharged using an AC charger 238 such as that illustrated in Figure 2B. In another embodiment an AC charger such as the charger 238 can be permanently connected to the energy source system in which case one of the compartments 220, 222 in the housing 204 can be adapted to receive the charger when not in use. This also allows an AC outlet to be used as a power source when such a power source is available to the user. In one embodiment, a non-portable respirator is contemplated in which, instead of portable energy sources such as the battery pack or battery cartridge, only an AC adaptor is provided for powering the system 200. While the embodiment of Fig 2 shows the power source compartments 220, 224 on either side of the blower portion of the housing, the power source could instead be located underneath the housing (the back side of the housing), however it will be appreciated that swapping out of battery packs or battery cartridges is made slightly more difficult in such an arrangement.

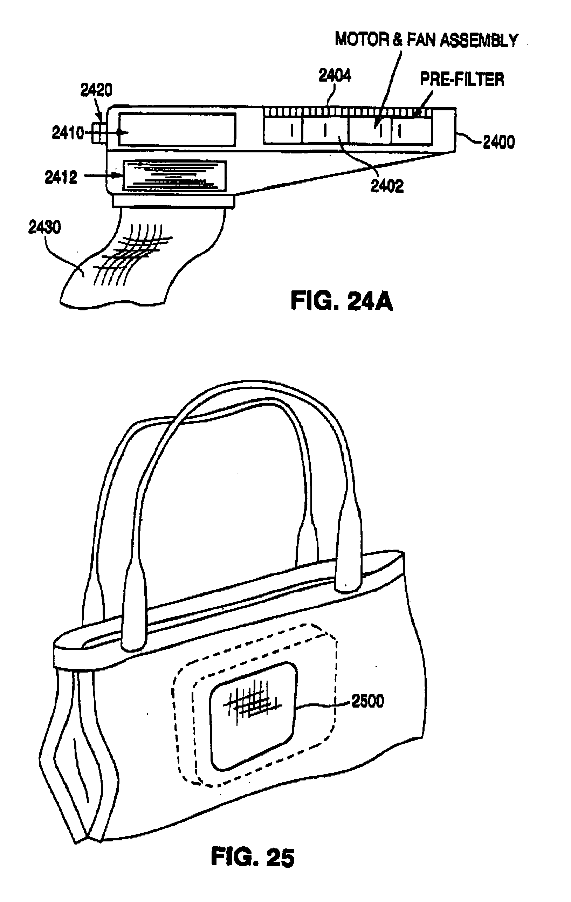

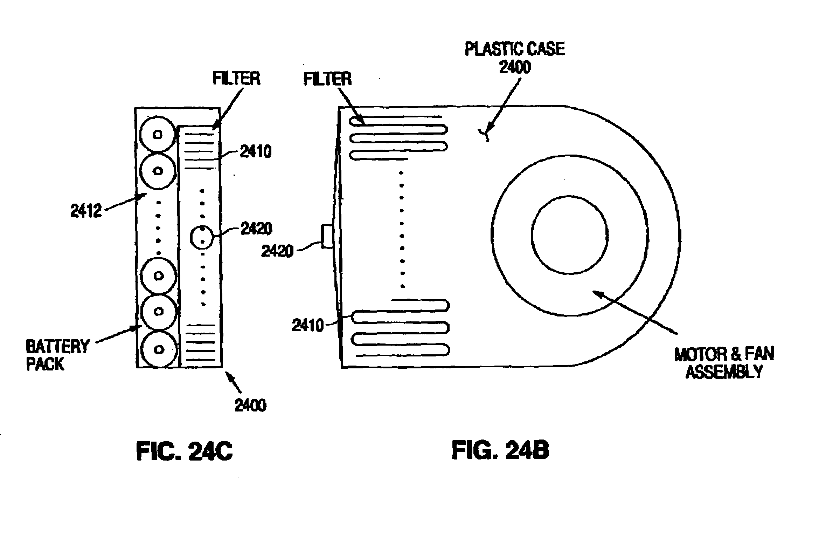

Another embodiment of the disclosed respirator is shown in Figures 24 A, B, C which show a housing 2400 with a centrifugal fan 2402 covered by a pre-filter 2404. The pre-filter prevents the fan 2402 and other components of the system from becoming clogged by large particles. The air from the fan is vented radial outwardly and is channeled by the housing wall through the main particle filter 2410, which is mounted above a battery pack 2412. The air is passed out of an outlet port 2410 to which a hose (not shown) is connectable. The housing with its blower, filter and power supply is attached in fanny-pack fashion by means of a belt 2430.

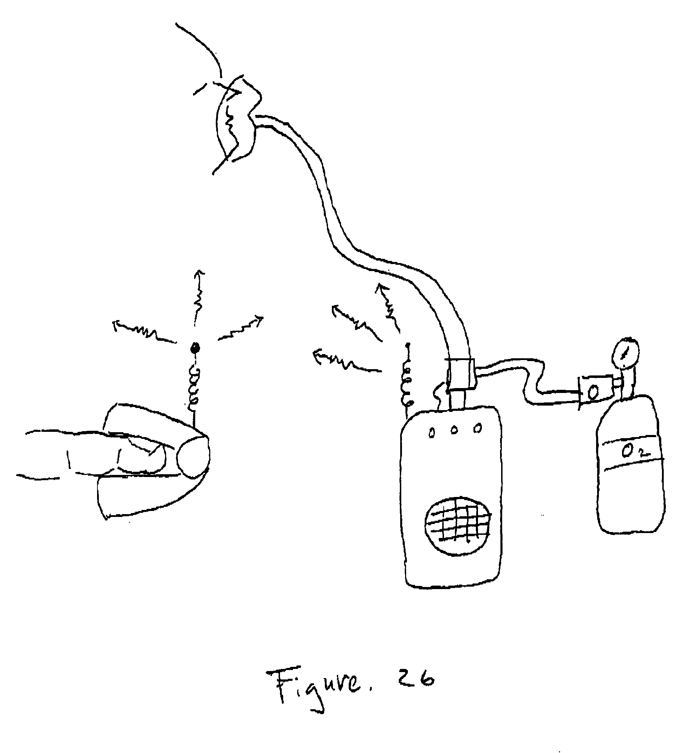

Considering again Figure 2A, this embodiment is also a portable system and the housing 205 is therefore also securable to a user. In this case it is secured to a belt 240 by providing a fanny-pack-type arrangement (not shown) or by providing a bag 250 sized to receive the housing 204 and having loops, clips, buttons or other attachment means for connecting the bag 250 to the belt 240. In certain embodiments, the disclosed respirator also proposes making the housing 204 or the bag or pack 250 carrying the housing 204, into a fashion item by providing the housing 204 or the pack or bag 250 with apparel designer trade dress as shown by the handbag arrangement shown in figure 25, which includes an air-permeable section 2500 for readily permitting air to be drawn in by the blower. The disclosed respirator also proposes providing swatches or replaceable covers for all or part of the housing 204 or pack or bag 250, thereby allowing users to swap out the look of the housing, pack or bag.

The disposable portion 205 of the system 200 comprises a particle filter 260 connected by means of a delivery hose or tube 262 to a face mask 264. The delivery tube 262 is connected directly to the filter 260 in this embodiment, however, in another embodiment the hose is connected to the housing 204, and the filter is located at the air outlet 210 or in the hose 262 or between the mask 264 and the hose 262 or in or partially in the face mask 264.

In order to make the delivery tube less unwieldy, the disclosed respirator proposes the use of a highly flexible hose with sufficient resistance to being crushed if the user presses on the tube. In one embodiment a flat rectangular hose having one or more channels extending through the hose is used. In another embodiment, as shown in Figures 2 and 3, a round cross-sectional highly flexible tube is used that gets its structural integrity from being either corrugated as shown from the side in Figure 4A and in cross-section in Figure 4B. Yet another embodiment simply involves a round cross-sectional hose with a sufficiently thick wall to resist being crushed. Another embodiment that involves a flattened cross-section is the oval cross-sectional, corrugated, flexible tube as shown from the top in Figure 5A, from the side in Figure 5B, and in cross-section in Figure 5C. For aesthetic reasons a transparent tube is proposed as an option irrespective of the configuration of the supply hose or tube. As a further aesthetic refinement, one embodiment provides a hose or tube that has similar styling to the face mask, *e.g.*, color and surface texture so that the hose becomes a natural extension of the mask. In fact, in one embodiment, the hose is shaped to attach to the mask in such a manner as to appear to be integrally formed with the mask. Typically, however, the hose will be formed separately for ease of manufacture, *e.g.,* by extrusion, while the mask can be formed by a molding process. To enhance the aesthetic appearance the disclosed respirator proposes the use of a tube that is smaller in diameter than prior art devices. In particular, a tube outer diameter of less than about 16 mm, *e.g.*, 10-16 mm is proposed. It will be appreciated that as the tube gets thinner, in order to provide the same flow rate, the air velocity has to be increased. One embodiment therefore proposes the inclusion of a baffle 300 in the face mask 264 to deflect the incoming air stream.
As mentioned above, the disclosed respirator makes use in one embodiment of a very small diameter hose of 10-16mm, which has not been done for breathing systems in the past. The disclosed respirator overcomes the flow problems of past systems by using the thin hose in conjunction with a much larger (3000 square centimeters or more) high quality particle filter (60-100 nm particle filter) thereby achieving a far less cumbersome system.

In another embodiment the disclosed respirator contemplates using a thicker outside diameter hose, *e.g.,* 20-25 mm or even larger in order to achieve very high flow rates for situations where high air consumption is involved. Nevertheless, in accordance with the disclosed respirator the hose is still used with the large filter area of 3000 square centimeters or more to optimize flow and reduce the amount of power consumed by the air mover. Thus it will be appreciated that the disclosed respirator derives one of its advantages by combining a large filter area with the appropriate diameter hose and adjusting blower power, thereby optimizing the energy consumption.

Another feature of the disclosed respirator, as indicated above, is the provision of disposable portions to the breathing apparatus. In one embodiment the hose, filter and mask are made disposable. This involves not only the releasability of the hose, mask and filter from the portion that is re-usable (in this case the housing with its air mover), but also the use of cheaper materials or thinner materials for the disposable mask and tube. In another embodiment, instead of the entire face mask being made to be disposable only the filter or filter cartridge can be made to be disposable and is thus removably connected to the mask.

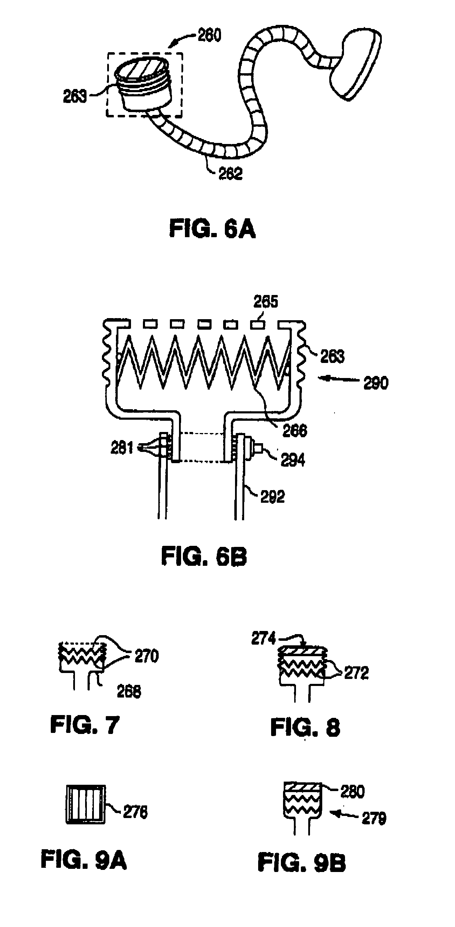

As mentioned above, the particle filter cartridge 260 can be connected to the hose 262, as shown in Figures 2 and 3 and in more detail in Figures 6A-6B. In this embodiment the particle filter is a ULPA filter with 0.1µm particle size filtering capability, the filter being implemented as a hockey puck-type arrangement of a standard size. The filter cartridge 260 has external threads 263 for screwing into an air outlet recess such as the outlet 210 of Figure 2A, which is provided with complementary threads to engage the threads 263. As is shown most clearly in the sectional view of Figure 6B, the cartridge 263 defines a housing with a molded gate or grill 265 over the top for retaining the filter material 266. A variation of the filter cartridge 262 is shown in figure 7 which shows a deeper housing 268 with larger diameter for accommodating two larger diameter filters 270. While the double filter arrangement provides greater resistance to airflow, the effect is addressed by providing the larger diameter. In yet another embodiment, shown in Figure 8, two particle filters 272 and a pre-filter 274 are provided as part of the filter cartridge. While the embodiments above all make use of circular filters, the disclosed respirator is not so limited. Instead of using a hockey puck arrangement for the filter cartridge, one embodiment makes use of a square filter 276 with 3-4 inch sides and corresponding housing 279, as shown in Figures 9A and 9B, respectively. It will be appreciated that rectangular filters could also be implemented and that multiple filters could be provided in a filter housing or filter cartridge similar to the Figures 7 and 8 arrangements. The filter cartridge 279 of this embodiment includes a pre-filter 280 having a function other than simple filtration. It makes use of an impregnated sponge, ring layer, or surface coating on a particle filter of manganese dioxide or copper oxide catalyst for converting carbon monoxide to carbon dioxide. In another embodiment, a solid ring layer impregnated with calcium hydroxide or calcium silicate solids removes sulfur dioxide and nitrogen dioxide. In yet another embodiment sulphur dioxide is instead removed by a wetted lime slurry in a sponge. In one embodiment ozone is removed using a honeycomb ceramic fiber, such as the Honeycycle ZG impregnated with active carbon or other means as previously described.

The advantage of a filter having a square or rectangular shape is that filter material is not wasted between filter cutouts, as is the case with a round filter. On the other hand the round filter provides the advantage that it makes the sealing process easier. Instead of round or square configuration filters, the filter can be annular (donut-shaped) and wrap around a blower in cases where the air from the blower is expelled radially outwardly. In such a configuration, the housing can form an annular channel around the filter and define an outlet on at least one side of the channel for connecting at least one hose.

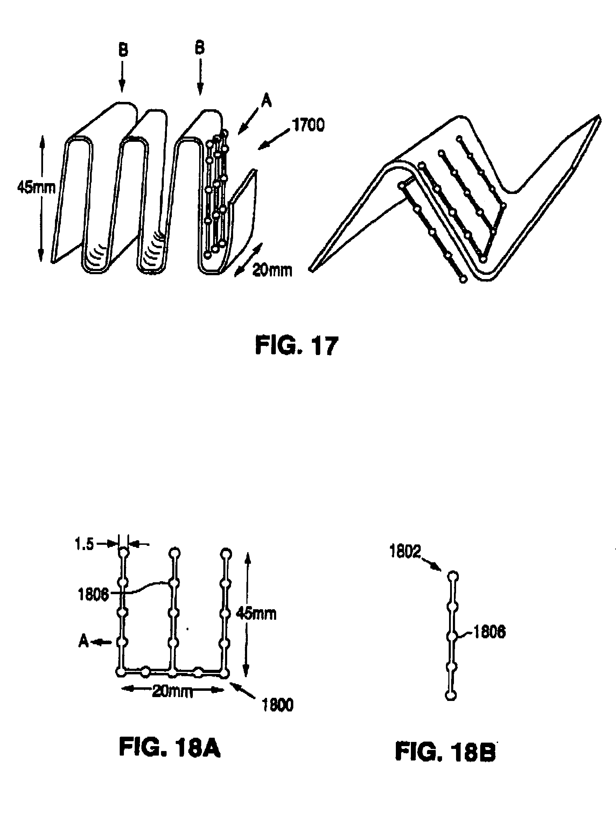

The filters themselves can be, and preferably are, pleated to increase the surface area. While the filter of Figure 6B is shown as having a zigzag configuration, a preferred embodiment is shown in Figure 17 which shows a pleated particle filter 1700 with rounded ends 1702. This rounded configuration is maintained by making use of spacers as shown in Figures 18 A and 18 B. Figure 18A shows a side view of a spacer 1800 that slots vertically (side 1802 at the top and 1804 at the bottom) into a pleat as indicated by letter A in Figures 17 and 18. The middle leg 1806 provides added support to the middle of the pleat to prevent it from collapsing. Since air flows through the filter in a direction indicated by arrows B, it will be appreciated that the spacer 1800 can not interrupt the air flow. This is achieved in this embodiment by providing the legs 1802, 1804, 1806 it a serpentine configuration thereby leaving vertically extending channels when inserted into the filter pleat. It will be appreciated that without departing from the scope of the disclosed respirator, other spacer configurations can be provided for providing rounded ends to pleated filters and for spacing the pleats apart while still permitting air to pass through the pleats. Also, it will be appreciated that rounded end pleats and spacers will also be usable in other filter applications and are not limited to portable respirators. For instance filtration systems in motor vehicles could be enhanced using this configuration and arrangement.

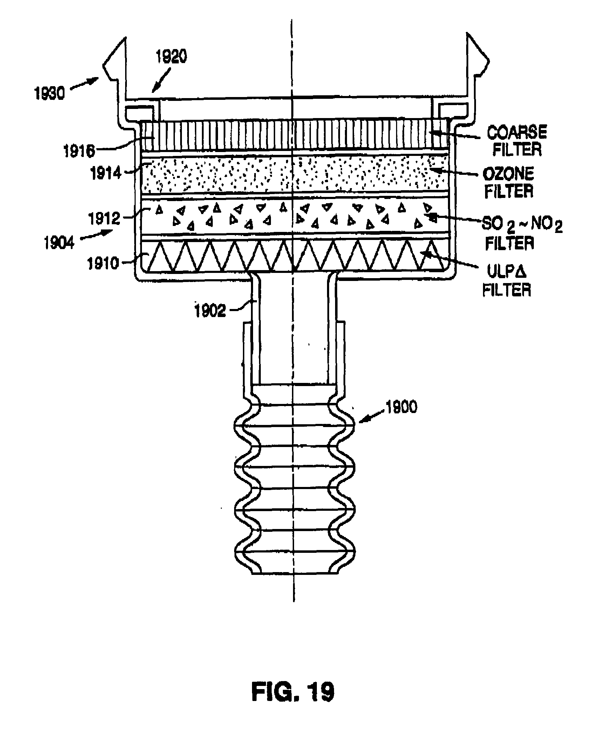

As mentioned above, the filters or the filter cartridges can be arranged to be easily removable to allow them to be disposed of and replaced with new filters or filter cartridges. In order to achieve this, the filter must be removably attached to the hose unless it is intended to discard the hose at the same time, in which case the hose can be permanently secured to the filter as shown by the configuration of Figure 19. The hose 1900 is connected to a plastic extension 1902 extending from the filter cartridge 1904. The cartridge in this embodiment includes not only the ULPA filter 1910, which forms the main filter, but also an SO2 and NO2 filter 1912, an ozone filter 1914, and a coarse particle filter 1916. The coarse particle filter 1916 is held in place by plastic fingers 1920 extending peripherally from the wall cartridge 1904. This embodiment comprises a square filter and is secured to a housing by slotting the cartridge 1904 into a complementary connector extending from the housing and securing it by means of flexible plastic clips integrally molded with the cartridge 1904 and engageable with complementary slots in the connector. It will be appreciated that the square connector that receives the cartridge 1904 must provide access to its slots to allow the clips or hooks 1930 to be pressed together in order to release the cartridge from the connector.

In another embodiment, instead of only the filter or the filter and hose being disposed of, the entire face mask with filter and the hose can be arranged to be disposable. In one embodiment, the filter, hose and face mask are designed to have different life expectancies, thereby requiring replacing after different periods of time.

The use of non-standard size hockey-puck configurations or any other non-standard configuration provides the additional benefit of capturing the replacement parts market.

While the particle filters discussed above could be any particle filters but preferably comprise submicron filters such as HEPA or ULPA filters capable of filtering out extremely small particles, *e.g.,* on the order of 60-100 nm.

The disclosed respirator also provides an advantageous placement of the particle filter relative to the other parts. In particular, the preferred embodiment places the particle filter 260 at the air outlet 210 of the housing 204. This defines a clean air zone on the downstream side of the filter 260 thereby avoiding contamination issues by the blower caused by having the blower located in the clean air zone. The addition of the pre-filter 212 in the housing provides some protection to the blower and electronics in the housing against dust and other larger particles. As an additional barrier against small particles, however, one embodiment of the disclosed respirator implements the pre-filter as a second HEPA or ULPA filter in the housing. As shown in Figure 19, in a preferred embodiment where one or more pre-filters are included in a filter cartridge, the main particle filter 1910 is placed on the downstream side to allow it to catch any debris from the pre-filters.

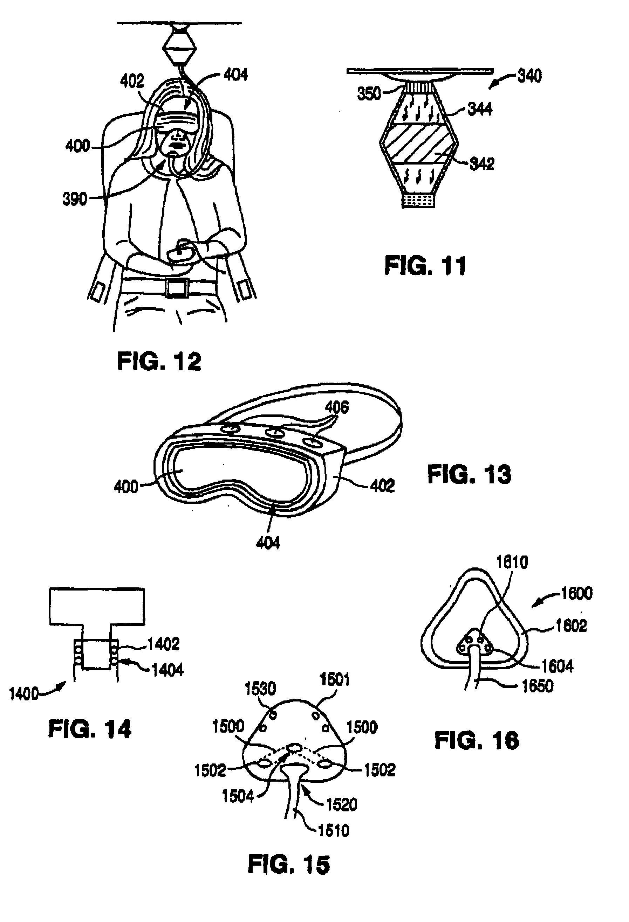

The face mask 264 of this embodiment has also been adapted for greater usability and aesthetic appeal by making it from a transparent material such as silicone. Such a mask 390 is shown in greater detail in Figure 12. In another embodiment only the mouth portion is made of a transparent material to allow the lips of the user to be observed during conversations. The wall thickness of the mask 390 in the mouth portion 392 is also reduced to cause less muffling of the user's voice. As discussed in our earlier, commonly assigned applications identified above, the mask can instead be provided with a microphone and speaker for facilitating conversations between the user and third parties.

In preferred embodiments, all joints or connections are designed to prevent the ingress of particles in excess of the filter capabilities of the particle filter 260. Connections between reusable parts or between replacement parts such as between the hose 262 and the face mask 264 and between the hose 262 and the filter cartridge 260 are done by way of permanent connections such as thermal adhesives or welds as in the case of the connection between the hose 1900 and the extension 1902 to the filter cartridge, which are connected by means of a thermal adhesive. In contrast, connections or seals between replacement parts and re-useable parts involve releasable connections such as one or more friction connectors, *e.g.,* O-rings or gaskets with multiple ribs. In the embodiment shown in Figure 6B, three O-rings 281 are used to connect the filter cartridge 290 to the hose 292, thereby providing built in redundancy. Figure 14 shows an embodiment of a hose connected to a filter cartridge by means of a seal 1400. The seal 1400 includes a multi-rib gasket defining a redundant layer seal in the form of two ring layers of sealant 1402 forming two ribs separated by a trough 1404. This multi-seal configuration reduces the likelihood of the integrity of the seal being compromised by mechanical stress, manufacturing defect, etc.

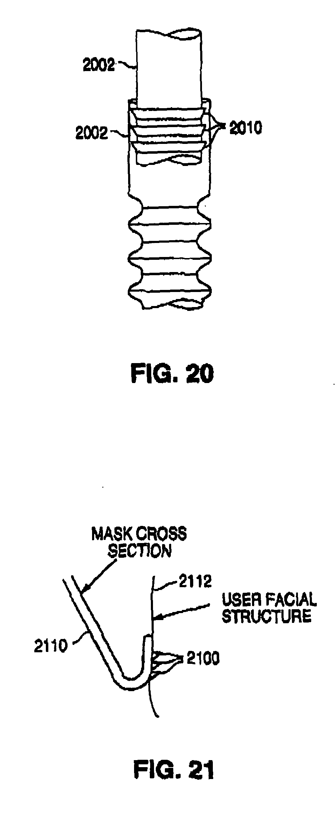

In the embodiment of Figure 20, a gasket, *e.g.,* made of a non-porous foam material, between the abutting surfaces is used. Figure 20 shows a hose 2000 slipped over a mask extension 2002. Multiple annular non-porous foam gaskets 2010 on the extension 2002 ensure a multiple redundancy seal between the hose 2000 and the extension 2002.

A gasket with multiple ribs defines a multiple, parallel seal 2100 along the periphery of the face mask 2110 to seal more effectively against the user's face 2112. In addition to the gaskets or seals, a clamp, such as the clamp 294 in Figure 6B can be used to ensure that the two parts do not easily come apart.

Some pre-filters for eliminating certain chemicals were discussed above. In addition, certain chemicals in the ambient air can be reduced by making use of surface coating and other forms of catalysts in the housing or supply hose. In order to reduce exposure to CO and ozone, one embodiment of the disclosed respirator includes a titanium coating on the inner surfaces of the housing 204 and hose 262 to act as catalyst for converting carbon monoxide (CO) to carbon dioxide (CO2). Other catalysts could also be used, *e.g.*, to split off an oxygen atom from the CO molecule to leave carbon and oxygen. It will be appreciated that only some of the surface area of the housing or hose can be provided with such a catalytic coating. In order to reduce ozone in the air a coating of manganese dioxide or copper oxide catalyst is provided on the inner surface of at least part of the housing 204 and supply hose 262 of one embodiment. In another embodiment sulfur dioxide and nitrogen dioxide are removed by a coating of calcium hydroxide or calcium silicate solids. In another embodiment sulfur dioxide is instead removed by a wetted lime slurry in a sponge. In yet another embodiment 03 is removed using a honeycomb ceramic fiber, such as the Honeycycle ZG impregnated with active carbon.

The embodiment of Figure 2 includes a pressure sensor and a controller for controlling power to the blower 260. An inlet valve 302 can be provided in the hose 262 (as depicted in FIG. 2A) or in the housing 204. The mask 264 also includes an outlet valve 304 to prevent outside, unfiltered air from entering the mask, and to facilitate a system that seeks to maintain a constant, slightly positive pressure in the mask as discussed above. The outlet valve 304 in one embodiment is implemented to open due to the added air pressure from the user's exhaled air. In order to avoid unfiltered surrounding air from entering the mask, one embodiment further includes at least one channel or conduit (in the embodiment of Figure 15, two channels 1500 are provided leading to outlet valves 1502) to maintain channels of clean air. The channels or conduits are arranged in the wall of the mask 1501 in this embodiment but could also be arranged along an inner or outer surface of the face mask. The air outlet 1504 in the mask of Figure 15 includes a split manifold in which air is split into two channels exiting the mask as two outlet ports 1502 each with its own outlet valve to provide for a more balanced and aesthetically pleasing mask. As shown in Figure 15, the air supply hose 1510 is arranged between the nose region (corresponding to the air outlet 1504) and the chin region 1520 and attaches to the mask in a way as to appear to be integrally formed with the mask. In this embodiment the hose is angled downwardly at an angle of 45 to 60 degrees from the perpendicular to provide a comfortable angle for a person working at a desk. In order to improve comfort to the user, the mask 1501 includes cross-air ventilation holes 1530, which are provided with high quality particle filters, *e.g.*, HEPA or ULPA filters to prevent unfiltered air from entering the mask.

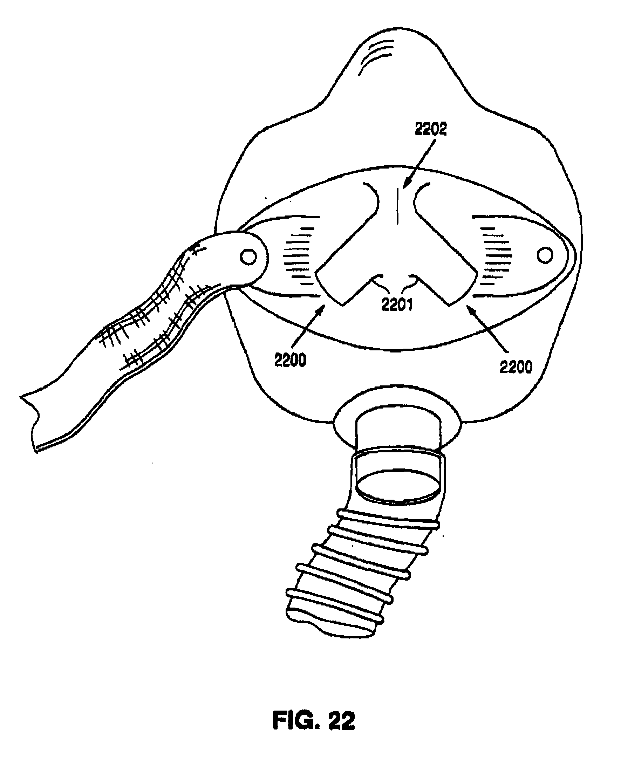

A similar configuration is shown in the mask of Figure 22 which also has dual channels 2201 extending to dual openings 2200 venting air to the outside. However, in this embodiment the outlet valve (not shown) is provided at the common internal opening 2202, while the external openings 2200 are simply outlet holes.

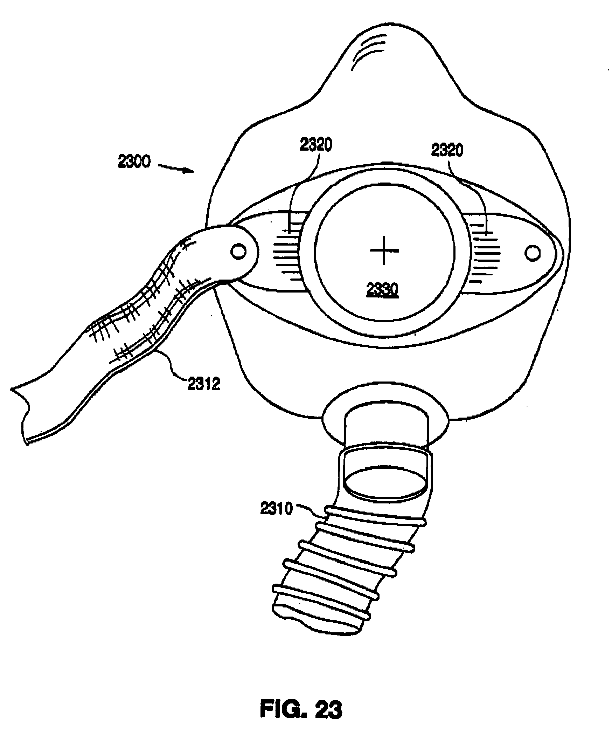

In another embodiment of a mask 2300 shown in Figure 23 with its hose 2310 and strap 2312, two outlet valves 2320 control air flow out of dual conduits so as to leave a central clear region 2330 to allow the user's mouth portion to be seen. The clear region in this embodiment is also made of a thinner material to improve communications by the user.

In another mask embodiment, a skin-like flexible mask body 1600 is provided as shown in Figure 16. The mask 1600 has a small rigid portion 1604 to provide an air space over the nose and mouth of the user. Also, the periphery of the mask is provided with a plurality of filter ribs arranged in parallel along the perimeter for greater integrity. As in the embodiment of Figure 15, the embodiment of Figure 16 includes cross-ventilation holes with HEPA filters 1610 in addition to the exit valve 1604 and a backup inlet valve for supplying clean air should the air flow via the air pipe 1650 be interrupted for some reason.

As mentioned above, the disclosed respirator, in certain of its more basic forms, contemplates implementation in a loosely fitting mask arrangement in which the mask does not seal to the face of the user but relies on the positive pressure to continuously expel filtered air thereby preventing unclean air from entering the mask. In this regard, as shown in Figure 2A, the face mask 264 can be provided with another particle filter 301 for allowing filtered air to continue entering the mask in the event that airflow from the housing 204 is somehow interrupted. Also, the face mask 264 provides a particle filter mounted at the outlet valve 304 to filter out particles from the exhaled air. In other embodiments, the face mask can be tight-fitting or substantially tight-fitting so as to substantially confirm to a user's face. In one embodiment of a tight-fitting mask, substantially all of the perimeter of the face touches a user's face and/or chin and/or jaw. It should be known that the particle filter mentioned above, in a preferred embodiment would be a sterilization chamber fabricated from materials such that the interior surfaces have a high reflectivity in the 250 nm to 280 nm wavelength range. The sterilization chamber utilizes ultraviolet light generated by mercury vapor lamp (lamps), light emitting diodes, or other light emitting opto-electronic devices (all such devices emitting UV radiation between 250 nm and 280 nm) to destroy the RNA or DNA of any airborn pathogens exhaled by the user.

The embodiment of Figure 2 contemplates using the re-usable portion 202 in conjunction with the disposable portion 205; however, in another embodiment, only the disposable portion 205 is used. In particular it is used with a public air supply system, *e.g.* an air supply system on an airplane, or in a bus, or train. In order to facilitate the connection of the disposable portion 205 to a public air supply system, the disclosed respirator makes use of a connector to allow interfacing the disposable portion 205 with the public air supply system. The connector will facilitate connection to one type of air supply system but not necessarily all such air supplies that are available. The disclosed respirator therefore also provides an adaptor for connecting to at least two but preferably all public air supply systems. One such adaptor 320 is shown in Figure 10. The adaptor 320 includes an adaptor housing 322 with a rubber annular seal 324 extending from one end to engage an air vent 330. A connector portion 312 at the opposite end of the housing 322 allows the inlet of the air supply hose to engage the housing 322 by way of a friction seal. The adaptor 320 provides a simple solution for attaching the disposable portion of the clean air system (particle filter, supply tube or hose, and face mask) to a public air supply system such as an air vent in an airplane.

Another embodiment of an adaptor 340 is shown in Figure 11, which includes the particle filter 342 inside a housing 344. The housing 344 is made from a flexible material that sealingly engages the air vent 350 of a public air supply system.

In the embodiment where the particle filter is connected to the inlet end of the hose, the adaptor is connected to the particle filter 260 instead of to the hose directly. On the other hand, if the particle filter is mounted in the hose 262, in the mask 264, between the outlet end of the hose 262 and mask 264, or in the adaptor itself as in the Figure 11 embodiment, the adaptor is connected directly to the inlet end of the hose 262.

While the above elements of the air supply apparatus have dealt with supplying clean air for breathing, the other aspect of the disclosed respirator is to provide a similar protection to the eyes. The disclosed respirator therefore includes a pair of goggles with filtered vent holes to facilitate the passage of filtered air into the mask. Thus the goggles are protected from fogging up while at the same time the user remains protected against unwanted exposure to contaminated air. One embodiment of such a pair of goggles is shown in Figure 13, which shows the back of the goggles that engages with the user's face. In this embodiment the goggles include a lens 400 supported in a frame 402. (For purposes of this disclosed respirator the term "lens" simply refers to the transparent glass or plastic viewing portion of the goggles and does not imply any concave or convex surfaces to the "lens".) The frame is held snugly against the user's face by a gasket or seal 404 secured along the periphery of the frame. The gasket 404 in this embodiment comprises three parallel gasket elements integrally made from a single piece of non-porous foam material to provide triple sealing or double redundant sealing. In addition, the gasket 404 is provided with filtered air vents 406 in the form of replaceable particle filters mounted in air holes in the gasket 404. This facilitates the air movement through the mask that avoids the goggles fogging up. The gasket 404 can also be implemented in a different way, *e.g*. by using a particle filter material instead of a non-porous foam material. In one embodiment the gasket is secured directly to the lens 400, *e.g.*, by gluing it to the lens, and avoiding the need for a frame altogether.

In one embodiment, the disclosed respirator can include a reserve reservoir to meet high peak inhalation airflow during sporadic exertion. The reservoir would temporarily supply pressurized air when the demand for air by the user exceeds the blower's capacity for a brief period.

In one embodiment, the PAPR provides a steady airflow of, for example, 350 slm. Occasionally, under high output conditions the user may have a peak inhalation air flow rate of up to 500 slm; however these tend to be spikes, and although they demand a very high flow, the duration is quite brief and hence the volume relatively small. For example, if the user is climbing a steep flight of stairs or riding a bicycle uphill. The reserve air reservoir draws down when the flow demand surpasses the capability of the blower and allows the user to continuously receive adequate flow.

The excess flow and pressure in the system supplied by the blower inflates the reservoir, for example during the user's exhale cycle. If the pressure in the system drops below a predefined point, the reservoir deflates, supplying the user with a pressurized flow, in a short time period, in addition to what the blower is capable of until it is empty. Once the pressure climbs to normal, the reservoir begins inflating again using excess flow and pressure.

There are several different configurations for situating the air reservoir in the disclosed respirators. For example, in one embodiment, the hose is used as the air reservoir. In this embodiment, pressure of the blower stretches the hose out, for example, from 75 cm to 90 cm for a reservoir of 74 mL (with a 25 mm hose). Pressure of the blower can inflate the hose, expanding the diameter from 25 mm to 30 mm for a reservoir of 161 mL. In one embodiment, a separate reservoir can be configured in line with the filter. For example, the air reservoir can be an air bladder which in one embodiment would have a capacity of 0.5 liters. However, it is understood that the bladder can be 0.1 to 2 liters in other embodiments designed in combination with a blower to provide the instantaneous air availability that is equivalent to respiratory demands that would otherwise require up to 1000 slm of continuous air flow. Using an air reservoir in combination with a blower has the added benefit of not requiring high flow rates through the exit valve during the non-peak portion of the respiratory inhalation cycle.

Air reservoirs can also include a small high pressure O₂ tank to provide a preset level of O₂ in the air, such as 30% instead of seal level atmospheric levels or can be used in conjunction with an oxygen sensor to maintain sea level atmospheric oxygen levels. In addition, the respirator embodiments disclosed herein may utilize a blood oxygen saturation sensor attached to the wearer's finger or other body part with a wired or wireless transmission to control flow rates, add O₂ or sound alarms for mask removal or filter change (as shown in Fig. 25).

While the disclosed respirator has been described with respect to specific embodiments, the disclosed respirator is not limited to these embodiments but can be implemented in different ways within the scope of the claims.

## Claims

1. A respirator apparatus, comprising:
an air mover operable to generate an ample amount of an air stream, the air mover having an air inlet and an air outlet;
a particle filter mounted in the air stream; and
a supply means operably connected and in fluid communication with the air outlet of the air mover,
wherein the respirator is configured to provide air to the supply such that users blood oxygen level remains relatively stable.

2. The respirator of claim 1, further comprising a face mask operably connected to the supply hose at an end opposite the supply hose end that is operably connected to the housing.

3. The respirator of claim 1, wherein the respirator is configured to provide air at a flow rate of at least approximately 350 slm.

4. The respirator of claim 1, wherein the respirator is configured to provide air at a flow rate of up to approximately 600 slm at a pressure up to 2.5 in H₂O.

5. The respirator of claim 4, wherein the air mover comprises an impeller of 10-cm diameter configured to operate at 5,000 rpm.

6. The respirator of claim 4, wherein the air mover comprises an impeller of 7-cm diameter configured to operate at 15,000 rpm.

7. The respirator of claim 1, wherein the particle filter comprises a high-efficiency particulate air (HEPA) filter material with 99.97% efficiency at 300-nanometer particle size.

8. The respirator of claim 1, wherein the particle filter comprises an ultra-low penetration air (ULPA) filter material with 99.999% efficiency at 25-nanometer particle size.

9. The respirator of claim 1, wherein the particle filter comprises a wet-laid glass media.

10. The respirator of claim 1, further comprising a power supply for the air mover, the power supply being disposed on the housing and comprising lithium-ion cells that deliver up to 14.4 V and have capacities of at least approximately 2.4 ampere-hour (Ah).

11. A system, comprising
an air mover operable to generate an air stream, the air mover having an air inlet and an air outlet;
a particle filter mounted in the air stream;
a supply hose operably connected at one end to the housing; and
an air reserve reservoir configured to provide a user of a system with additional air without increasing power to the air mover.

12. The system of claim 11, wherein the air reserve reservoir is configured as the supply hose, wherein the supply hose stretches to accommodate additional air until the additional air is needed by the user.

13. The system of claim 11, wherein the air reserve reservoir is a separate air bladder that is in line with the particle filter.

14. The system of claim 11, wherein the system is configured to provide an air supply of at least 200 standard liters per minute (slm).

15. A respirator apparatus, comprising
a housing;
an air mover mounted in the housing, the air mover being operable to generate an air stream, the air mover having an air inlet and an air outlet;
a particle filter mounted in the air stream, wherein the particle filter comprises a wet-laid glass media; and
a supply hose operably connected at one end to the housing.

16. The respirator of claim 15, wherein the particle filter comprises a high-efficiency particulate air (HEPA) filter material with 99.97% efficiency at 300-nanometer particle size.

17. The respirator of claim 15, wherein the particle filter comprises an ultra-low penetration air (ULPA) filter material with 99.999% efficiency at 25-nanometer particle size.

18. The respirator of claim 15, further comprising a power supply for the air mover, the power supply being disposed on the housing and comprising lithium-ion cells that deliver up to 14.4 V and have capacities of at least approximately 2.4 ampere-hour (Ah).

19. A respirator apparatus, comprising
an impeller operable to generate an air stream at a speed of at least 5,000 rpm, and the impeller having an air inlet and an air outlet;
a particle filter mounted in the air stream; and
a supply hose operably connected at one end to the housing.

20. The respirator of claim 19, wherein the impeller comprises a 10-cm diameter.

21. The respirator of claim 19, wherein the impeller comprises a 7-cm diameter and operates at a speed of approximately 15,000 rpm.
